# EUROPEAN PATENT APPLICATION

(11) **EP 3 456 414 A1**
(43) Date of publication of application: **20.03.2019**
(21) Application number: 17191863.4
(22) Date of filing: 19.09.2017
(51) Int. Cl.: B01L 3/00, C12Q 1/68, G01N 27/447, G01N 33/487

(54) **SETUP AND METHOD FOR THE CAPTURE OF A BIOPOLYMER AND ITS CONTROLLED TRANSLOCATION THROUGH A NANOPOROUS NETWORK**

(71) Applicant: Siemens Healthcare GmbH, 91052 Erlangen (DE)
(72) Inventor: Paulicka, Peter, 91341 Röttenbach (DE); Gumbrecht, walter, 91074 Herzogenaurach (DE)

(57) **Abstract**

The present invention relates to a setup for the capture of at least one biopolymer and its controlled translocation through a nanoporous network, a method of capturing at least one biopolymer and translocating it through a nanoporous network using the setup, as well as sub-arrays and arrays with a multitude of these setups together with tunneling electrodes, at least one read-out circuit and at least one detector for sequencing the biopolymers.

## Description

The present invention relates to a setup for the capture of at least one biopolymer and its controlled translocation through a nanoporous network, a method of capturing at least one biopolymer and translocating it through a nanoporous network using the setup, as well as sub-arrays and arrays with a multitude of these setups together with tunneling electrodes, at least one read-out circuit and at least one detector for sequencing the biopolymers.

Technologies and methods for the sequence determination of nucleic acids experienced a revolutionary development during the last 10 years. Quality improved, while costs dramatically decreased.

Nevertheless the technologies are still dominated by biological concepts such as sequencing by synthesis, making use of enzymes, e.g. polymerases, to create new nucleic acid strands, complimentary to the analyte strands (e.g. from Illumina, IonTorrent, PacBio). Several technologies are dependent on e.g. optical labels (e.g. from Illumina, PacBio). Read lengths are still too short.

Strand sequencing by feeding the target nucleic acid molecules through nanopores may overcome the read-length problem, but current technologies are still dependent on biological membranes, pore-proteins and enzymes to control the translocation-velocity of the nucleic acid-strand (e.g. from Oxford Nanopore). These biological components suffer from complex manufacturing steps, sample preparation efforts and shelf-life issues.

Already 1999, an "All Solid State and Label-Free" concept has been proposed in US 6,627,067 B1, the transverse tunneling method, which is shown as a basic concept in Fig. 1. As shown in the figure, an electrical tunneling current (Iₜᵤₙₙₑₗ) through the bio-molecule, forced by a transversal voltage Vₜ, is detected, while the biopolymer is translocated perpendicularly through the tunneling electrode junction, driven by a longitudinal voltage Vₗ. As can be further seen from the figure, three different resistances have to be considered for the longitudinal voltage, namely the electrical resistances for the electrolyte cavities (R_{E}) and for the nanopore (R_{N}), which will be discussed afterwards. The feasibility of discriminating the different nucleic acid (NA) bases by different tunneling currents has been shown in e.g. Ohshiro T, Matsubara K, Tsutsui M, Furuhashi M, Taniguchi M, et al. (2012) Single-molecule electrical random resequencing of DNA and RNA; Sci Rep 2: 501 and Di Ventra M. (2013) "Fast DNA sequencing by electrical means inches closer"; Nanotechnology 24: 342501.

Beside the technical issues of manufacturing sub-nanometer pores with aligned nanometer electrodes, the reliable, noise-reduced readout of base-specific electrical currents is one of the major issues to bring this concept into products. A critical restriction hereby is the bandwidth-limitation of the tunneling-current amplifier, requiring a translocation-velocity limitation of nucleic acid when passing the electrode-junction. This could be achieved by a reduction of the longitudinal voltage Vₗ.

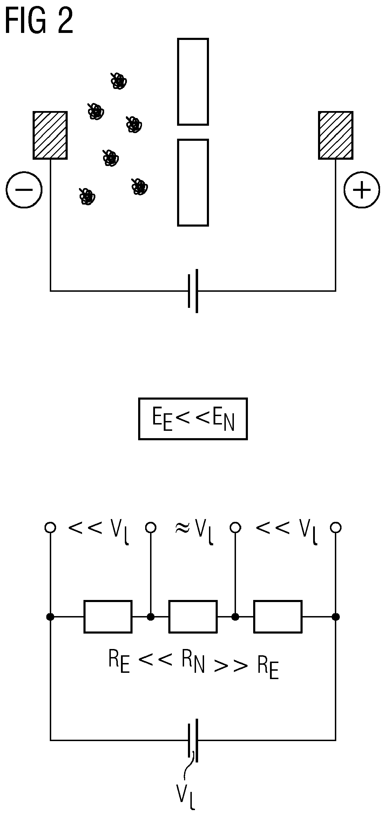

In a typical setup for transverse tunneling, two electrolyte-cavities are separated by a membrane, showing one exemplary nanopore, as depicted in Fig. 2. Fig. 2 therein shows exemplary DNA translocation in a longitudinal field trough a single nanopore, as well as the electrical resistances associated therewith. The constellation of the electrical resistances for the electrolyte cavities (R_{E}) and for the nanopore (R_{N}) shows that almost the complete voltage Vₗ drops inside the nanopore, providing a very high electrical field (E_{N}), which induces a very high translocation-velocity. But due to the negligible electrical fields (E_{E}) inside the electrolyte cavities, there is almost no electrophoretic force to move the nucleic acid molecule to the entrance of the pore, which causes inacceptable delays in a sequencing process.

In other words, a fast capture and slow translocation is needed, but a slow capture and fast translocation is provided in the state of the art discussed above.

This situation is partly improved by the introduction of nanoporous networks instead of single nanopores, as disclosed in e.g. WO 2014048816 A1 and WO 2015010904 A1 and shown schematically in Fig. 3.

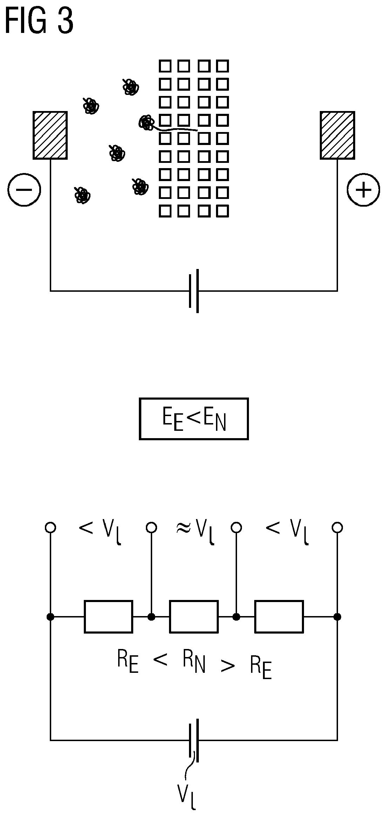

Fig. 3 therein shows the DNA translocation in a longitudinal field trough a nanoporous network, wherein an improvement in the relative electrical resistances is achieved by the setup provided with a nanoporous network.

But both approaches, single pores, as well as nanoporous networks, show the contradiction of the needs to provide high longitudinal voltages to attract nucleic acid strands to the tunneling junction inlet and low longitudinal voltages to provide moderate translocation inside the tunneling junction.

Therefore there exists a need to provide an improved setup for capturing and translocating biopolymers through a nanoporous network.

Particularly a task of the invention is to provide a solution for this contradiction, which is compatible to the transverse tunneling concept, can preferably be integrated into a CMOS-chip platform, and can realize an efficient workflow in production of a setup as well as during a use thereof in capturing and translocating biopolymers.

### Summary of the Invention

The inventors realized that a basic problem with the approach of the state of the art is that both the capturing and the translocating of biopolymers are carried out with the same setup in one step. Thus, the basic idea for the proposed solution to the above problem is to de-couple both processes, the attraction/capturing (catch) and translocation to provide dedicated voltages for each.

In a first aspect the present invention relates to a setup for the capture of at least one biopolymer, preferably at least one nucleic acid molecule, and its controlled translocation through a nanoporous network, comprising:
- a flow channel on a solid substrate configured to introduce a solution or suspension of the at least one biopolymer;
- at least two main electrodes on the solid substrate inside the flow channel; and
- a, e.g. continuous, nanoporous network layer in the flow channel on the solid substrate adjacent to and/or on at least one of the two main electrodes;
wherein the flow channel is configured to introduce the at least one biopolymer at a location on an opposite site of at least one of the two main electrodes in relation to the nanoporous network layer.

A second aspect of the present invention relates to a method of capturing at least one biopolymer, preferably at least one nucleic acid molecule, and its controlled translocation through a nanoporous network, comprising:
- providing the setup of the first aspect of the invention,
- providing a solution or suspension containing the at least one biopolymer,
- introducing the solution or suspension containing the at least one biopolymer into the setup of the first aspect of the invention,
- applying a suitable voltage between at least two electrodes of the setup of the first aspect of the invention for a time sufficient to introduce the at least one biopolymer into the nanoporous network layer,
- replacing the solution or suspension with a hydrophobic liquid, and
- applying a suitable voltage between the at least two main electrodes to translocate the at least one biopolymer inside the nanoporous network layer.

A third aspect of the present invention is directed to a pre-subarray, comprising a multitude of setups of the first aspect of the invention and at least one readout-circuit.

Also disclosed in a fourth aspect is a pre-array, comprising a multitude of pre-subarrays of the third aspect.

A fifth aspect relates to a device for sequencing at least one biopolymer, comprising
the setup of the first aspect of the invention;
a pair of tunneling electrodes; and
a detector for detecting a tunneling current through the pair of tunneling electrodes.

In a sixth aspect a sub-array for sequencing at least one biopolymer is disclosed, comprising
the pre-subarray of the third aspect;
a multitude of pairs of tunneling electrodes, wherein for each setup of the first aspect of the invention a pair of tunneling electrodes is provided; and
a detector for detecting tunneling currents through the multitude of pairs of tunneling electrodes.

A seventh aspect relates to an array, comprising a multitude of sub-arrays of the sixth aspect, or comprising
a pre-array of the fourth aspect;
a multitude of pairs of tunneling electrodes, wherein for each setup of the first aspect of the invention a pair of tunneling electrodes is provided; and
at least one detector for detecting tunneling currents through the multitude of pairs of tunneling electrodes.

Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description they serve as explanation of concepts and principles of the invention. Other embodiments and many of the stated advantages can be derived in relation to the drawings. The elements of the drawings are not necessarily to scale towards each other. Identical, functionally equivalent and acting equal features and components are denoted in the figures of the drawings with the same reference numbers, unless noted otherwise.
Fig. 1 shows the basic transverse tunneling concept known from the state of the art.
In Fig. 2 a schematic setup for transverse tunneling in the state of the art is depicted.
Fig. 3 shows the DNA translocation in a longitudinal field trough a nanoporous network according to the state of the art.
Figs. 4 to 6 show an abstract approach of a capture and translocation of a biopolymer using the present setup.
Figs. 7 to 9 show the capture and translocation of a biopolymer using a certain embodiment of the present setup as well as an exemplary method of the present invention.
Figs. 10 to 12 show the capture and translocation of a biopolymer using a further certain embodiment of the present setup as well as an exemplary method of the present invention.
Figs. 13 and 14 show another setup and method according to the invention.
Fig. 15 shows a basic planar concept for transverse tunneling, with V_{c} and Vₜ alternatively applied.
Fig. 16 depicts the planar concept of Fig. 15 with a nanoporous layer thereon.
Fig. 17 shows a cross-section of a tunneling junction in the setup of Fig. 16 in view of the arrow depicted therein, perpendicular to the translocation direction.
In Fig. 18 an exemplary subarray of the present invention is presented schematically.
Figs. 19 to 22 show schematically the production of the present setup.
Fig. 23 depicts schematically an exemplary setup, an exemplary subarray and an exemplary array of the present invention.
In Figs. 24 and 25 the capture of a biopolymer using an exemplary setup of the invention is presented.
Figs. 26 and 27 show the capture of exemplary biopolymers in two exemplary setups of the present invention.
Figs. 28 to 30 show an exemplary device of the present invention as well as the use thereof for detecting biopolymers.
In Figs. 31 to 34 the translocation of DNA as an exemplary biopolymer as well as the guiding thereof to a pair of tunneling electrodes for detection is presented.
Figs. 35 and 36 show an exemplary translocation of DNA as an exemplary biopolymer using the present setup in a method of the invention.

### Detailed description of the present invention

### Definitions

Unless defined otherwise, technical and scientific terms used herein have the same meaning as commonly understood by one of ordinary skill in the art to which this invention belongs.

A biopolymer is a polymer produced by a living organism, i.e. a polymeric biomolecule. The term particularly includes particularly proteins and/or nucleic acid molecules.

The term "nucleic acid molecule" refers to a polynucleotide molecule having a defined sequence. It comprises DNA molecules, RNA molecules, nucleotide analog molecules and combinations and derivatives thereof, such as DNA molecules or RNA molecules with incorporated nucleotide analogs or cDNA. It also comprises cell free (cf) DNA and RNA. The term "nucleic acid sequence" relates to the sequence of nucleotides in the nucleic acid molecule. In the present description, the terms "nucleic acid molecule" and "nucleic acid" can be used interchangeably, unless clear from context that they should be differentiated.

A "capture" of a biopolymer relates to the introduction of the biopolymer, at least partially and preferably in whole, into a nanoporous network layer, particularly at a location near one main electrode that can in such instance be configured as a capture electrode. The capture of the biopolymer can particularly be carried out by applying an electrical field that directs the biopolymer into the nanoporous network layer, e.g. by applying an electric potential to one of the main electrodes in the present setup that attracts the at least one biopolymer, e.g. a positive potential in the case of nucleic acid molecules. For a capture, it is not necessary that the at least one biopolymer gets into contact with a main electrode.

A "translocation" of the biopolymer relates to the movement of the biopolymer within the nanoporous network layer e.g. from a location where it is captured to a different location, e.g. to a pair of tunneling electrodes. During this movement, the biopolymer can particularly be stretched so that it can be easily sequenced afterwards. The translocation of the biopolymer can be carried out by applying an electrical field that "moves "the biopolymer through the nanoporous network. This electrical field for translocating the biopolymer particularly is different from the electrical field for capturing the biopolymer with regard to electrical field strength and/or the direction of the electrical field, i.e. the direction of the vectors of the electrical field, at least in some locations of the electrical field, particularly a location where at least one biopolymer is present. This can be e.g. done by changing the electric potential at the main electrode that captured the at least one biopolymer, e.g. to a negative potential in the case of a nucleic acid molecule, and applying an opposite potential to another, e.g. the second or further, main electrode, e.g. a positive potential in the case of nucleic acid molecules. For the translocation, it is also possible to use more than one main electrode to attract the at least one biopolymer from the main electrode that captured the at least one biopolymer, so that e.g. more than one biopolymer can be directed towards different main electrodes during translocation, so that the biopolymers can be brought to e.g. different tunneling electrodes for detecting and sequencing the biopolymers. This is particularly useful if not all tunneling electrodes for sequencing are working with the same efficiency, so that e.g. a subarray with more than two main electrodes and more than a pair of tunneling electrodes can be tested for the most efficient tunneling electrode.

In the present invention, also the term "nanoporous layer" can be used in place of the term "nanoporous network layer", so that the two terms can be used interchangeable, unless clear from context that something different is meant. The term "nanoporous network" refers to the network

In the present invention, all numerical values are considered to be complemented by the term "about".

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

The present invention relates in a first aspect to a setup for the capture of at least one biopolymer, preferably at least one nucleic acid molecule, and its controlled translocation through a nanoporous network, comprising:
- a flow channel on a solid substrate configured to introduce a solution or suspension of the at least one biopolymer;
- at least two main electrodes on the solid substrate inside the flow channel; and
- a, e.g. continuous, nanoporous network layer in the flow channel on the solid substrate adjacent to and/or on at least one of the two main electrodes;
wherein the flow channel is configured to introduce the at least one biopolymer at a location on an opposite site of at least one of the two main electrodes in relation to the nanoporous network layer.

In the present setup, the two main electrodes are not particularly restricted. They can comprise or be of any material, e.g. a metal like Pt, Cu, W, Ag, AgCl, Au, Al, etc., a conductive ceramic, a conductive polymer, mixtures thereof, etc., and can be of any dimension as long as they are big enough to attract a biopolymer to them and a sufficient voltage can be applied to them. The voltage can be applied through one or more vias, e.g. 2, 3, 4, 5, 6, 7, 8, 9 or more vias, through the substrate to the main electrodes, meaning that more than one via can be used per electrode. The main electrodes can have any shape and can be e.g. produced by applying a suitable photo mask for their production, e.g. having a rectangular, longitudinal, round, square, elliptic, etc. shape. They can also be just represented by the surface of one or more, e.g. 2, 3, 4, 5, 6, 7, 8, 9, 10 ore more vi-as, e.g. if a planar surface for forming the nanoporous network layer is intended. Furthermore, the thickness of the main electrodes is not particularly restricted as long as they can be covered by the nanoporous network layer. For example, the main electrodes can have a square or round shape with dimensions, i.e. length or diameter, in a range between about 0.05 to about 100 µm, e.g. about 0.1 to about 10 µm. A suitable thickness may be between about 0.01 and about 100 µm, e.g. between about 0.1 and about 10 µm. The at least two main electrodes can also vary in shape and/or size between each other or be the same. As indicated, it is also possible that the main electrodes, as well as possibly one or more auxiliary electrodes described later, do not extend beyond the surface of the solid substrate, i.e. that there is essentially no height difference between the electrodes and the solid substrate, so that the continuous nanoporous network layer can be formed on top of the solid substrate comprising the main and optionally auxiliary electrodes.

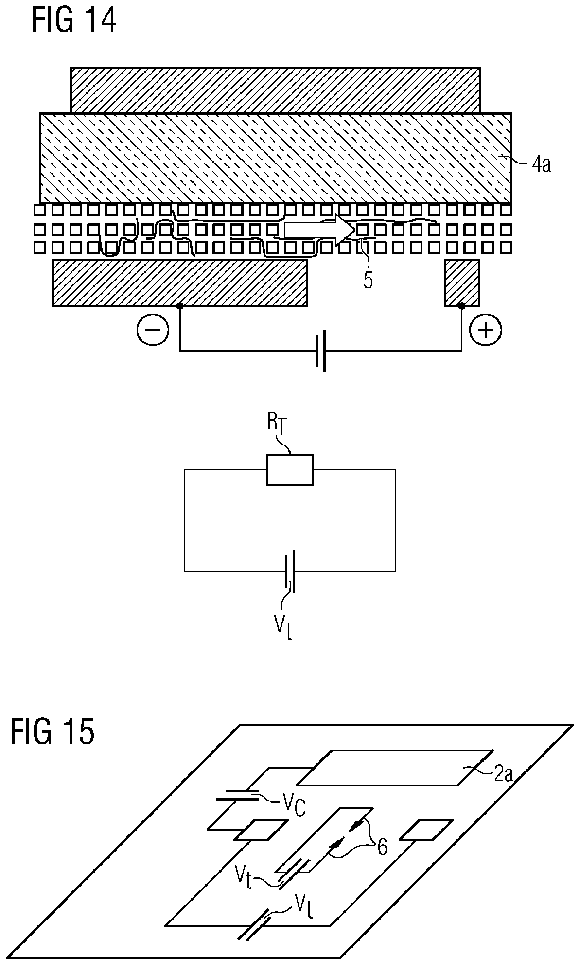

Further, also the solid substrate is not particularly restricted. According to certain embodiments, the solid substrate is essentially flat. According to certain embodiments, the solid substrate is a silicon-based or glass-based chip, e.g. also a silicon wafer or based thereon, preferably a CMOS- (complementary metal-oxide-semiconductor) chip. The present setup and method are especially suitable to be integrated onto a silicon-chip with e.g. CMOS circuitry, because both electrical processes, the electrophoresis-based translocation process as well as the tunneling process, can be performed in a planar lateral way, as can be also seen in Figs. 15, 16, 17 described later. This means that it is not excluded that the substrate itself comprises parts of an electric circuit, e.g. vias as discussed above, and/or even a circuit to contact the electrodes provided thereon. The substrate can also comprise several layers, but also can comprise only one layer.

In addition, the flow channel that is configured to introduce a solution or suspension of the at least one biopolymer is not particularly restricted as long as it is configured to introduce the at least one biopolymer at a location on an opposite site of at least one of the two main electrodes in relation to the nanoporous network layer. It can be of any suitable material, e.g. glass, plastic, etc. The dimensions of the flow channel can be adapted to the size of the solid substrate. In the present invention, it is not excluded that the flow channel is provided over a pre-subarray, a pre-array, a subarray or an array, if more than one setup is provided on a solid substrate.

Also the nanoporous network layer is not particularly restricted and can be made of any material, e.g. of conductive and/or insulating particles, as e.g. described in WO 2014048816 A1 and WO 2015010904 A1. According to certain embodiments, the nanoporous network layer is made of an essentially non-conducting or insulating material, e.g. Al₂O₃, TiO₂, etc. It can be produced by any suitable method, e.g. a sol-gel-process, using e.g. a metal alcoholate precursor solution, generating a sol-solution of nano-particles with e.g. a diameter of about 0.1 to about 100 nm, e.g. about 0.5 to about 50 nm, e.g. about 1 to about 10 nm, subsequently converting into a gel, which can be tempered to create the corresponding nanoporous network. The involved viscous liquid materials can be applied e.g. by spin-coating. The finalized nanoporous layer can be structured e.g. by standard photolithography and etching. The nanoporous network layer is characterized in that it forms a network of nanopores in which the at least one biopolymer can be captured and through which the at least one biopolymer can be translocated, i.e. can be moved through more than one, e.g. several, pores. It thus differs from nanopillars which do not form nanopores, but nano-slits at most.

According to certain embodiments, the nanoporous network layer is a continuous nanoporous network layer that covers at least both main electrodes. However, it is not excluded that only one main electrode is covered if this electrode is used for capturing and the nanoporous network layer extends in such a way that a translocation of a biopolymer therein after capture, e.g. to a pair of tunneling electrodes, is possible. Also, it is possible that one or both of the main electrodes are placed adjacent to the continuous nanoporous network layer on one side thereof, e.g. if the nanoporous network layer separates the at least two main electrodes from a place where the solution or suspension of the at least one biopolymer is introduced. Of course also mixed embodiments, e.g. with one main electrode covered by and a second main electrode adjacent to the nanoporous network layer, are possible.

According to certain embodiments the nanoporous network is hydrophilic so that an aqueous solution or suspension of at least one biopolymer can be introduced more easily into it and/or can be absorbed thereby.

The thickness of the nanoporous network layer is not particularly restricted and can be e.g. about 10 to several 100 nm, e.g. about 10 to about 1000 nm, e.g. about 20 to about 400, 500, 600, 700 or 800 nm.

The nanopores in the nanoporous network layer are not particularly restricted with regard to their shape as long as some of the pores therein have a diameter of less than 1 µm, i.e. are nanopores. According to certain embodiments, the nanoporous network layer comprises nanopores with a diameter of about 0.5 to about 900 nm, preferably about 1 to about 100 nm, further preferably smaller than about 10 nm, considering a DNA thickness of about 1 nm. The pores in the nanoporous network layer can be e.g. represented roughly by the pores in a closest package if the nanoparticles forming the nanoporous network layer are essentially round, wherein e.g. the pores represent about 25 vol.% of the total volume.

According to certain embodiments, the present setup further comprises at least one auxiliary electrode, which can be placed on the substrate, i.e. coplanar with the at least two main electrodes (aside the main electrodes), or on an opposite side of the at least two main electrodes in relation to the nanoporous network layer inside the flow channel, e.g. above the at least two main electrodes and the nanoporous network layer (adversely placed to the main electrodes). The size of the at least one auxiliary electrode as well as the shape thereof is not particularly restricted and can be the same as one of the main electrodes, or can be different. It can e.g. have a rectangular, longitudinal, round, square, elliptic, etc. shape. Furthermore, the thickness of the at least one auxiliary electrode is not particularly restricted, and can e.g. have a square or round shape with dimensions, i.e. length or diameter, in a range between about 0.05 to about 100 µm, e.g. about 0.1 to about 10 µm. A suitable thickness may be between about 0.01 and about 100 µm, e.g. between about 0.1 and about 10 µm. If more than one auxiliary electrode is present, the auxiliary electrodes can differ in shape and/or size or be the same.

When at least one auxiliary electrode is placed coplanar with the at least two main electrodes, the manufacturing of the setup is easier. In this case it is preferred that a gap exists between the at least one auxiliary electrode and the nanoporous network layer for introducing the solution or suspension of the at least one biopolymer. The size of the gap is not restricted as long as the solution or suspension of the at least one biopolymer can be introduced, and can be e.g. between about 0.01 and about 100000 nm, e.g. between about 1 and about 10000 nm.

When at least one auxiliary electrode is placed on an opposite side of the at least two main electrodes in relation to the nanoporous network layer inside the flow channel, an electrical field applied between at least one of the two main electrodes and the at least one auxiliary electrode can be easier adjusted and controlled. According to certain embodiments, the at least one auxiliary electrode is not in contact with the nanoporous network layer and particularly not covered by the nanoporous network layer. Again, a gap is preferred for introducing the solution or suspension of the at least one biopolymer. The size of the gap again is not restricted as long as the solution or suspension of the at least one biopolymer can be introduced, and can be e.g. between about 1 µm and 1 mm, e.g. between about 50 and about 500 µm.

When at least one auxiliary electrode is present, it can be used for the capture of the at least one biopolymer, e.g. by applying a voltage between one of the main electrodes and the at least one auxiliary electrode, so that the at least one biopolymer is attracted by the one main electrode. Afterwards, the voltage can be applied between the main electrodes for translocation, with the voltage at the at least one auxiliary electrode switched off.

Therefore, if at least one auxiliary electrode is present, the present setup for the capture of at least one biopolymer, preferably at least one nucleic acid molecule, and its controlled translocation through a nanoporous network, can comprise:
- a flow channel on a solid substrate configured to introduce a solution or suspension of the at least one biopolymer;
- a, e.g. continuous, nanoporous network layer in the flow channel on the solid substrate,
- at least two main electrodes on the solid substrate inside the flow channel,
- at least one auxiliary electrode inside the flow channel, e.g. on the solid substrate or on an opposite side of the at least two main electrodes in relation to the nanoporous network layer inside the flow channel,
wherein the flow channel formed between the at least one auxiliary electrode and the continuous nanoporous network layer and configured to introduce the solution or suspension of the at least one biopolymer at a location on an opposite site of at least one of the two main electrodes in relation to the nanoporous network layer.

According to certain embodiments, at least one main electrode and at least one auxiliary electrode are placed on opposite sides of the continuous nanoporous network layer, e.g. facing each other. According to certain embodiment, at least one main electrode and at least one auxiliary electrode are facing each other. According to certain embodiments at least one of the at least two main electrodes and the at least one auxiliary electrode are placed on opposite sides of the nanoporous network layer facing each other. According to certain embodiments the at least two main electrodes, optionally the auxiliary electrode and the nanoporous network layer are placed coplanar on the substrate. According to certain embodiments, the at least two main electrodes, the at least one auxiliary electrode and the nanoporous network layer are placed coplanar on the substrate.

Furthermore, the present setup can comprise means to provide the solution or suspension containing the at least one biopolymer to the flow channel. This means to provide the solution or suspension can, according to certain embodiments, be configured to provide the solution or suspension in such a way that the solution or suspension contacts the nanoporous network layer and optionally - according to certain embodiments - the optional one or more auxiliary electrode. The means to provide the solution or suspension can be e.g. a further flow channel, e.g. if the setup is placed in a micro-fluidic device, e.g. a microchip or lab-on-a-chip, a tube, a capillary, etc.

According to certain embodiments, the present setup comprises a geometrical constellation to provide an electrical resistance R_{C} inside the nanoporous network layer between at least one main electrode, which can function as catcher electrode, and the solution or suspension of the at least one biopolymer, e.g. an electrolyte containing the at least one biopolymer, which is low enough to generate suitable electrical fields (E_{E} of the solvent - e.g. further comprising electrolyte(s) and/or buffer(s) - of the solution or suspension, e.g. electrolyte, E_{C} of the catcher electrode) between the catcher electrode and a second main electrode or an auxiliary electrode to move the at least one biopolymer from the electrolyte into the nanoporous network layer.

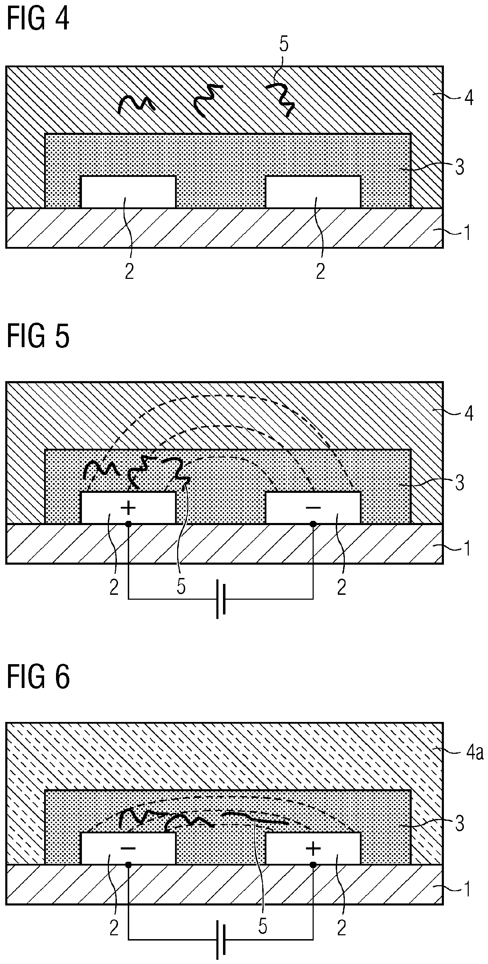

Figs. 4 to 6 show an abstract approach of a capture and translocation of a biopolymer using the present setup. According to Fig. 4, an exemplary pair of main electrodes 2 is provided on the solid substrate 1, and a nanoporous network layer 3 covers both main electrodes 2. On this structure, a solvent 4, e.g. an aqueous liquid which can comprise further components like buffers and/or electrolytes, etc., comprising biopolymers 5, e.g. nucleic acid molecules, is introduced, and the solvent 4 can enter the nanoporous network layer 3(not shown in detail).

In Fig. 5 it is shown how the biopolymers 5, which are in this case negatively charged, as e.g. nucleic acid molecules are, are attracted to one of the main electrodes to which a positive electrical potential is applied, e.g. in Fig. 5 on the left, by application of a first electrical field between the two main electrodes 2. The biopolymers 5 are thus captured inside the nanoporous network layer 3.

After the capture, the aqueous solvent 4 is exchanged by a hydrophobic liquid 4a and the biopolymers 5 are translocated through the nanoporous network layer 3 by application of a second electrical field between the two main electrodes 2. This second field differs in geometry and field strength so that the biopolymers 5 stay inside the nanoporous network. layer 3. Further, the polarity of the main electrodes 2 is reversed so that the biopolymers 5 translocate to the right electrode. The exchange of the liquid ensures that the biopolymers do not leave the nanoporous network layer, e.g. by the introduction of an oil, that hinders the biopolymers 5 from leaving the nanoporous network layer 3.

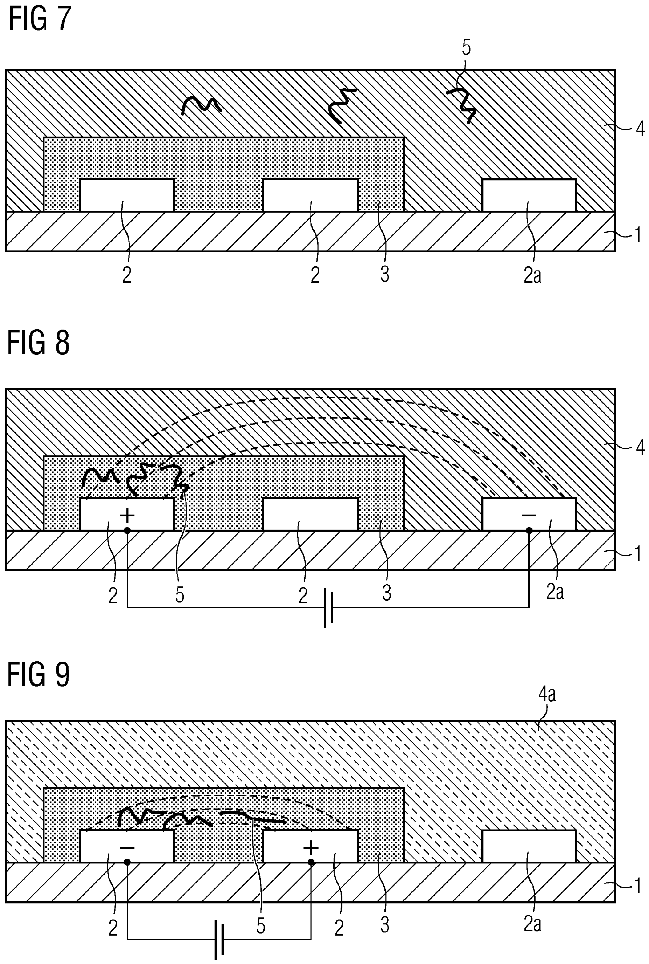

A further abstract concept for capture and translocation of biopolymers using a setup of the present invention is shown in Figs. 7 to 9. Figs. 7 to 9 show the capture and translocation of a biopolymer using a certain embodiment of the present setup as well as an exemplary method of the present invention wherein an additional auxiliary electrode 2a is provided coplanar with the two main electrodes 2 and the nanoporous network layer 3 on the solid substrate 1. As shown in Fig. 7, a gap exists between the auxiliary electrode 2a and the nanoporous network layer that can be filled with the solvent 4, or - as shown in Fig. 9 - with a hydrophobic liquid 4a.

As shown in Fig. 8 the capturing electrical field is in this case applied between one main electrode 2 (on the left) and the auxiliary electrode 2a, so that the biopolymers are introduced into the nanoporous network layer 3 near the main electrode 2 that is connected as anode. In this regard it is noted that it is also not excluded that for capturing an electrical field can be applied between more than one main electrode, e.g. two main electrodes, and one or more auxiliary electrode. For example, an electrical field between two or more, e.g. two, main electrodes, which both can attract the biopolymer to be captured, and one or more, e.g. one, auxiliary electrode with an opposite potential so that biopolymers can be captured on the two or more, e.g. two, main electrodes at the same time. During translocation biopolymers on e.g. one main electrode will then be translocated, while they may stay at another, e.g. the second, main electrode in a first translocation step. However, as a translocation can be done in both directions, and as a repeated back and forth translocation between main electrodes can be of advantage for detecting and sequencing the biopolymer, this can be remedied by carrying out more than one translocation process.

Again, after the capture, the aqueous solvent 4 is exchanged by a hydrophobic liquid 4a, and the biopolymers 5 are translocated through the nanoporous network layer 3 by application of an electrical field between the two main electrodes 2 and switching off the electrical field between the one main electrode 2 and the auxiliary electrode 2a present during capture (Fig. 8). This is shown in Fig. 9, wherein again the left main electrode 2 is used as cathode and the other main electrode 2 as anode to attract the biopolymers 5 through the nanoporous network layer 3. Again, the hydrophobic liquid 4a can hinder the biopolymers 5 from leaving the nanoporous network layer 3.

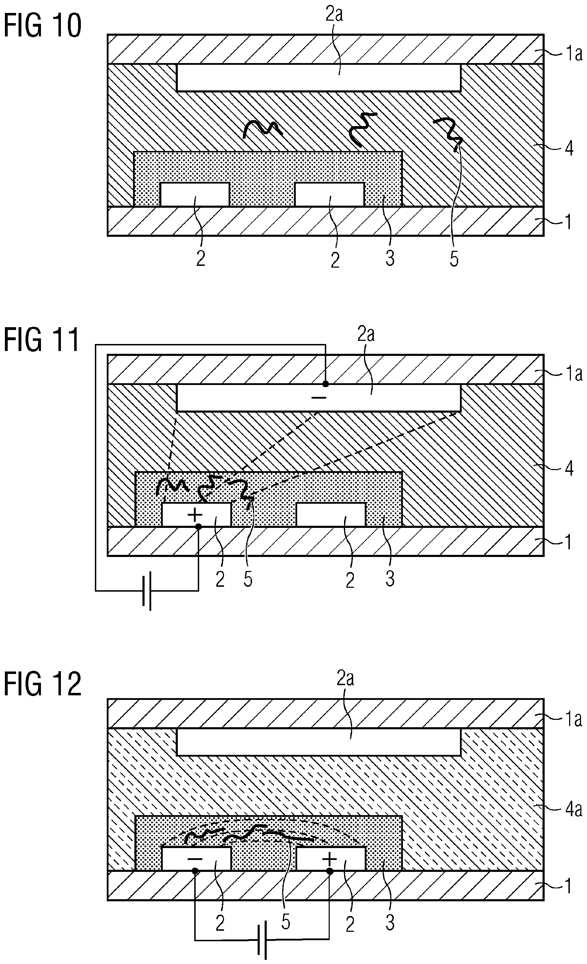

Figs. 10 to 12 show the capture and translocation of a biopolymer using a further certain embodiment of the present setup as well as an exemplary method of the present invention with a different setup using an auxiliary electrode 2a, which in this case is applied on a surface 1a of a flow channel on an opposite side of the two main electrodes 2 in relation to the nanoporous network layer 3 inside the flow channel, here above the two main electrodes 2 and the nanoporous network layer 3. As shown in Fig. 11, capturing is carried out again by applying an electrical field between the auxiliary electrode 2a and the left main electrode 2 so that the biopolymers 5 are caught in the nanoporous network layer 3 near the left electrode 2. Again, a hydrophobic liquid 4a replaces the solvent 4, as shown in Fig. 12. Further the translocation is shown again in Fig. 12, similar to Fig. 9, by applying an electric field between the main electrodes and switching off the electrical field shown in Fig. 11.

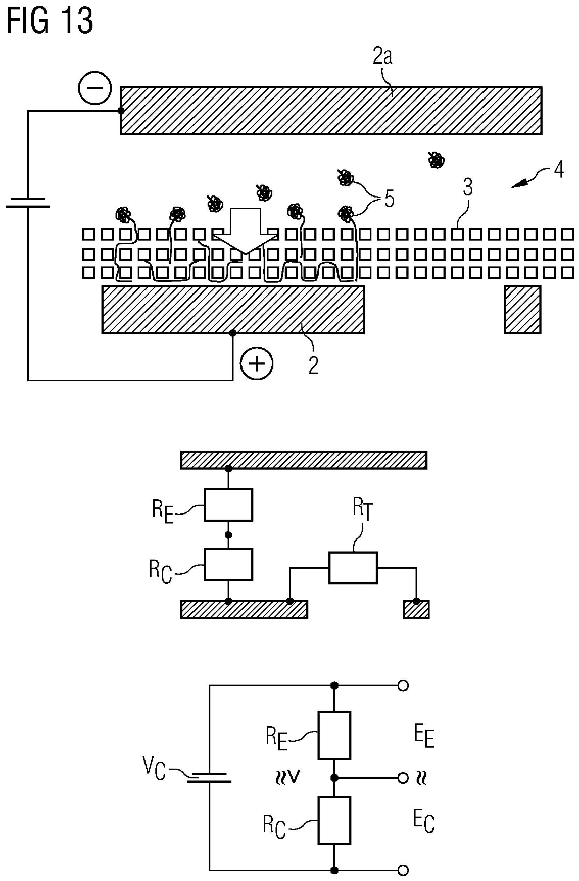

Figs. 13 and 14 show another setup and method according to the invention, which also represents the different steps shown in Figs. 11 and 12 in more detail.

As shown in Fig. 13, the biopolymers 5 are attracted to the main electrode 2 on the left by applying an electric field between the main electrode 2 on the left and the auxiliary electrode 2a into the nanoporous network layer 3. As further shown, this setup provides three different resistances for the biopolymers 5, namely the resistance R_{E} of the solvent 4, the resistance R_{C} for capture inside the nanoporous network layer 3, and a resistance R_{T} for translocation with R(E) ≈R(C) <R(T). In the setup shown, R_{E} is about the same or slightly higher than R_{C} so that the capture is enhanced compared to the state of the art, and the electrical fields in the solvent 4 (E_{E}) and in the nanoporous network layer (E_{C}) are similar when a capture voltage V_{C} is applied. The geometrical constellation in this figure thus provides an electrical resistance R_{C} inside the nanoporous network layer between the at least one main electrode 2 on the left which functions as catcher electrode and the solvent 4 which is low enough to generate suitable electrical fields (E_{E} of the solvent, e.g. electrolyte, E_{C} of the catcher electrode) between the catcher electrode 2 and the auxiliary electrode 2a to move the biopolymer 5 from the solution into the nanoporous network layer 3.

Fig. 14 then depicts in more detail how the biopolymers 5 are elongated by applying the longitudinal voltage Vₗ for translocating the biopolymers 5 through the nanoporous network layer 3, which represents a resistance Rₜ, which is thus also the resistance that has to be overcome to translocate the biopolymers to e.g. tunneling electrodes for detection and sequencing. The hydrophobic liquid 4a represents a further resistance to the biopolymers 5 so that they do not leave the nanoporous network layer.

Thus, these figures show in certain embodiments how a method of the present invention can be carried out using the present setup.

In a second aspect the present invention therefore relates to a method of capturing at least one biopolymer, preferably at least one nucleic acid molecule, and its controlled translocation through a nanoporous network, comprising:
- providing the setup of the first aspect,
- providing a solution or suspension containing the at least one biopolymer,
- introducing the solution or suspension containing the at least one biopolymer into the setup of the first aspect,
- applying a suitable voltage, e.g. of about 0.01 to about 4V, e.g. about 0.05 to about 3 V, e.g. about 0.08 to about 2.5 V, e.g. about 0.1 V to about 2 V, between at least two electrodes of the setup of the first aspect for a time sufficient to introduce the at least one biopolymer into the, e.g. continuous, nanoporous network layer, e.g. a time of several seconds to a few minutes, e.g. of 2 s to 10 mins, e.g. 5 s to 5 mins, e.g. 10s to 4 mins, e.g. 20 s. to 3 mins,
- replacing the solution or suspension with a hydrophobic liquid, and
- applying a suitable voltage, e.g. of about 0.001 to about 4V, e.g. about 0.005 to about 3 V, e.g. about 0.008 to about 2.5 V, e.g. about 0.01 V to about 2 V, between the at least two main electrodes to translocate the at least one biopolymer inside the nanoporous network layer.

With regard to applying a suitable voltage between at least two electrodes of the setup of the first aspect, particularly with regard to the application of a suitable voltage between the at least two main electrodes to translocate the at least one biopolymer inside the nanoporous network layer, it should be noted that the applied voltage will depend on the nature of the biopolymer to be captured and translocated, further constituents of the solution or suspension containing the at least one biopolymer, e.g. a solvent, the material of the electrodes, etc.

For example, when water is used as a solvent, an upper limit of about 2 V for the applied voltage may be of advantage to avoid electrolysis of water, e.g. when metals like Pt or Au are used as electrode material or other materials that can lead to the development of radicals and a formation of gas due to water electrolysis. When other electrode materials like Cu or Ag/AgCl are used, ions like Cu²⁺ or Cl⁻ can be formed, so that a formation of radicals and gases can be avoided when using water as a solvent, so that also higher voltages can be applied.

Also it should be noted that of course also higher voltages, e.g. above 2 V, can be applied for a short time, e.g. in a pulsed method, if necessary, even if water is used as a solvent.

The application of the voltage between at least two electrodes of the setup of the first aspect for a time sufficient to introduce the at least one biopolymer into the, e.g. continuous, nanoporous network layer can comprise, as shown above, the application of a voltage between at least two main electrodes, as e.g. shown in Figs. 4 to 6, or the application of a voltage between at least one main electrode and at least one auxiliary electrode - if present, as e.g. shown in Figs. 7 to 9, 10 to 12, and 13 and 14. A suitable voltage and a sufficient time thereby can be set according to the solvent used in the solution or suspension, the at least one biopolymer, the material of the nanoporous network layer and the pore sizes therein, etc. According to certain embodiments, the at least two electrodes for introducing the at least one biopolymer into the, e.g. continuous, nanoporous network layer can be thus the at least main two electrodes; or they can be represented by one of the two main electrodes and at least one, e.g. one, auxiliary electrode if it is contained.

Also 3 or more electrodes can be involved for capturing. For example, in case of nucleic acids as biopolymers, one negatively polarized auxiliary electrode and two positively polarized main electrodes may be used to capture the nucleic acids with both main electrodes, as also described above. For translocation, e.g. the two main electrodes are involved, keeping the captured nucleic acid above one electrode more or less stationary in a first translocation step. After inversion of the polarization, all nucleic acid molecules can be translocated into the opposite direction, e.g. to the other main electrode.

A suitable voltage between the at least two main electrodes to translocate the at least one biopolymer inside the nanoporous network layer can be set according to the solvent used in the solution or suspension, the at least one biopolymer, the material of the nanoporous network layer and the pore sizes therein, etc., in order to provide a desired velocity for translocating the at least one biopolymer, e.g. for detection and or sequencing, e.g. through a pair of tunneling electrodes. It can be e.g. between about 0.001 and about 4 V, e.g. between about 0.005 and about 3 V, e.g. between about 0.008 and about 2.5 V, e.g. between about 0.01 V and about 2 V. For example, the voltage for the translocation can be set lower than the voltage for capture of the at least one biopolymer.

From the foregoing, the present method, in other words, comprises:
- providing the setup of the present invention,
- providing a suspension or solution of at least one biopolymer, e.g. an electrolyte solution containing at least one biopolymer, to the setup,
- applying a suitable catch voltage (V_{c}) between two electrodes, e.g. between at least two main electrodes or between at least an auxiliary electrode and at least a main electrode (catcher-electrode), for a certain time to attract a sufficient amount of the biopolymer, e.g. strands, from the solution or suspension into the nanoporous network layer, e.g. above at least one main electrode, and store the captured biopolymer inside the nanoporous network layer,
- replacing the electrolyte solution by a non-aqueous, hydrophobic liquid, e.g. an oil like mineral-oil or silicone-oil, keeping the captured biopolymers in a solvent environment, e.g. an aqueous environment, inside the nanoporous network layer, and
- applying a suitable longitudinal voltage (Vₗ) between at least two main electrodes to translocate the at least one biopolymer inside the nanoporous network layer from the catcher-electrode to a second main electrode with a desired velocity.

The present setup and method allow the application of a sufficiently high catch voltage V_{c}, e.g. between about 0.01 and about 4 V, e.g. between about 0.05 and about 3 V, e.g. between about 0.08 and about 2.5 V, e.g. between about 0.1 and about 2 V, to capture the target biopolymers from the solution or suspension, e.g. electrolyte solution, in a considerable short time even when the concentration is very low. In principle it can be possible to collect all target biopolymer molecules from a desired solvent volume, which can e.g. be about 1 to about 1000 µL, e.g. about 10 to about 100 µL.

In conjunction with the nanoporous network layer, the biopolymer, for example a nucleic acid molecule, e.g. folded single stranded DNA or RNA, can be forced to unfold when entering the nanoporous network. The setup during the capturing process can be adjusted to keep the electrical field inside the solvent of the solution or suspension, e.g. an electrolyte, similar to the electrical field inside the nanoporous network layer. This can be e.g. adjusted by a suitable ratio of electrolyte thickness, e.g. inside the flow channel, versus nanoporous layer thickness and the area of the catcher electrode. The thickness of the nanoporous layer is not particularly restricted and can be e.g. between 5 nm and 5 µm, e.g. between 8 nm and 2 µm, e.g. between 10 nm and 1 µm, e.g. between 20 nm and 500 nm, e.g. between 50 nm and 200 nm, and can be e.g. about 100 nm. The area thereof is not particularly restricted as well and can be e.g. between 0.1 and 1000 µm², e.g. between 0.5 and 500 µm², e.g. between 1 to 100 µm², e.g. between 2 and 50 µm², e.g. between 5 and 20 µm², and can be e.g. about 10 µm².

For the purpose of translocating the biopolymer through the nanoporous network layer and, in the end, optionally passing a detector, like a tunneling electrode junction, with a well-defined velocity, the voltage Vₗ can be chosen sufficiently low e.g. between about 0.001 and about 4 V, e.g. between about 0.005 and about 3 V, e.g. between about 0.008 and about 2.5 V, e.g. between about 0.01 V and about 2 V, in the present method.

The solution or suspension containing the at least one biopolymer is not particularly restricted and can contain, apart from the at least one biopolymer, e.g. at least one nucleic acid molecule, and at least one solvent, e.g. water or a mixture of water with a further solvent or a non-aqueous solvent or a mixture of non-aqueous solvents, further components that are not particularly restricted, e.g. one or more electrolytes which are not particularly restricted and which can support the biopolymer capture and translocation, one or more buffers, and/or substances which can manipulate the at least one biopolymer, e.g. which support the formation of single stranded nucleic acid, such as formamide, etc. The solution or suspension can be composed to provide a dedicated, typically low ionic-strength to optimize the electrophoretic transport of the biopolymer, e.g. by introduction of zwitterion-buffers, such as histidine, and/or a dedicated pH to stabilize the at least one biopolymer, e.g. nucleic acid or protein. The solution or suspension can be of natural or artificial origin and can comprise e.g. a patient sample or a solution or suspension derived from a patient sample, e.g. a solution or suspension that is worked up from a patient sample and/or where further components, e.g. electrolytes and/or buffers, are added. In the solution or suspension a non-aqueous solvent is not particularly restricted and can be e.g. an alcohol, e.g. ethanol, propanol, phenol, glycol, etc., an ester, etc.

According to certain embodiments, a solvent for the solution or suspension containing the at least one biopolymer is water.

In this regard a "sample" is a sample containing at least one biopolymer to be captured and translocated, e.g. at least a nucleic acid molecule. The sample is not particularly restricted and can be of natural or synthetic origin and can e.g. comprise samples from simple or complex matrices, e.g. from a subject, e.g. a vertebrate, e.g. a human or animal, or from a natural source, e.g. an extract from soil, air, or a natural water/water body. The sample can also be water-free or water-poor, e.g. when it contains a non-aqueous solvent.

In a preferred embodiment the sample can comprise nucleic acid molecules, and the method is used to provide extracted, cleaned nucleic acid (e.g. DNA), particularly in a solvent of low ionic strength, e.g. a Histidine buffer of e.g. 10 mM, e.g. with an additive like formamide to stabilize ssDNA (single strand DNA).

In a further embodiment, the at least one biopolymer, e.g. nucleic acid, can be bound to a magnetic beads, e.g. magnetic silica beads, which then can be captured in the setup, e.g. above the nanoporous network layer, e.g. just above the electrodes, or the "electrode system" as a whole, by a magnetic field which can be applied to the setup. The captured beads with the at least one biopolymer then can be washed with a suitable solvent, and then the solvent can be exchanged with a suitable compound or compound mixture to further prepare the biopolymer for capture. E.g. a solvent like water for washing can be exchanged with a low ionic-strength, histidine and formamide containing buffer in case DNA is the at least one biopolymer to be captured.

Also, further pre-capture processes can be carried out, e.g. a temperature change in the setup. For example, supported by an intermediate temperature increase, nucleic acid as a biopolymer can be deliberated from the magnetic beads, denatured by formamide and finally captured into the nanoporous layer by the present, electrophoretic, capturing process.

The magnetic beads can e.g. remain when the hydrophobic liquid is introduced as they should not interfere with the translocation, or can be washed out before the translocation.

Using magnetic beads, the volume to be handled in the present method can be reduced, so that the method can be carried out more efficient.

According to certain embodiments the sample is an aqueous sample, i.e. a sample containing water. For example, the sample can be a body fluid of a subject. Body fluids are thereby liquids originating from inside the bodies of subjects, particularly living subjects. They include fluids that can be excreted or secreted from the body and/or that circulate in the body, and body water. They can be in the state of a liquid, emulsion or suspension. Examples of body fluids within the invention are blood, urine, saliva, sputum, plasma, serum, etc. According to certain embodiments, the sample is a patient sample (clinical isolate). Exemplified samples are serum, plasma, and/or whole blood of a patient.

According to certain embodiments, the subject in the present method of extracting a substance from a sample, enriching a substance in a sample and/or detecting a substance in a sample can be a vertebrate, preferably a human or animal, more preferably a mammal and most preferred a human, respectively human patient.

A vertebrate within the present invention refers to animals having a vertebrate, which includes mammals - including humans, birds, reptiles, amphibians and fishes. The present invention thus is not only suitable for humans and the human medical field, but also for veterinary medicine.

According to certain embodiments, the hydrophobic liquid is an oil, e.g. a mineral oil and/or a silicon oil. The mineral oil and the silicon oil are therein not particularly restricted. The mineral oil and or silicon oil can hinder the biopolymer(s) from leaving the nanoporous network layer and restricts the electrical translocation field within the nanoporous layer so that the translocation of the biopolymer(s) through the nanoporous network layer is enhanced, thereby providing for a good linearization of the biopolymer(s), i.e. for stretching the at least one biopolymer from e.g. a ball-like or irregular shaped form into a linear form. The translocation therein does not necessarily have to result of a translocation of the whole biopolymer at the same time and can e.g. also relate to a translocation of only a part thereof, e.g. one end thereof, in a certain direction, followed by a translocation of further parts thereof at a later time. It is also not necessary during translocation that the whole biopolymer is brought into linear form as long as the biopolymer can be translocated to a certain goal, e.g. a pair of tunneling electrodes, so that it then can pass there through with a defined direction.

The replacement of the solvent of the solution or suspension, e.g. an electrolyte by a hydrophobic liquid, e.g. oil further can avoid an electrical short-circuit during translocation as well as a bypassing of biopolymers of a tunneling electrode junction.

A third aspect of the present invention is directed to a pre-subarray, comprising a multitude of setups of the invention, i.e. of the first aspect, and at least one readout-circuit.

A multitude of setups therein can include 2, 3, 4, 5, 6, 7, 8, 9, 10, 11, 12, 13, 14, 15, 16 or more setups, which can all be also on one solid substrate, i.e. include a setup with one solid substrate on which the electrodes - main and optionally auxiliary electrodes - and nanoporous network layers are combined. It is also not excluded that the multitude of flow channels that can be formed in such a subarray can form one flow channel itself, e.g. by connecting a multitude of flow channels. Also the nanoporous network layers can be interconnected, e.g. for an ease of manufacturing.

The readout-circuit in the pre-subarray is not particularly restricted and can be configured to connect suitably the electrodes provided. It can also connect additional vias which can be present and or further electrode bases and/or electrodes, e.g. for forming tunneling electrodes and/or reference electrodes. In the present invention, it is possible to form a subarray from the present pre-subarray by e.g. forming pairs of tunneling electrodes on the substrate, e.g. on vias and optionally electrode bases that are already formed in the pre-subarrays. Therefore it is also possible to provide a pre-subarray on which then pairs of tunneling electrodes are formed in a later step, e.g. at a customer's site.

Also, a detector for detecting tunneling currents on a pair or multiple pairs of tunneling electrodes to be formed can already be provided in the present pre-subarray according to certain embodiments, which is not particularly restricted and which can comprise those known in the state of the art, e.g. from WO 2014048816 A1 and WO 2015010904 A1.

In the pre-subarray, the multitude of setups is preferably located on one substrate, in which e.g. the at least one readout-circuit can be integrated.

Also disclosed is in a fourth aspect a pre-array, comprising a multitude of pre-subarrays of the third aspect.

Again, a multitude of pre-subarrays therein can include 2, 3, 4, 5, 6, 7, 8, 9, 10, 11, 12, 13, 14, 15, 16 or more pre-subarrays, which can all be also on one solid substrate, i.e. include a setup with one solid substrate on which the electrodes - main and optionally auxiliary electrodes - and nanoporous network layers are combined. It is also again not excluded that the multitude of flow channels that can be formed in such an array can form one flow channel itself, e.g. by connecting a multitude of flow channels. Also the nanoporous network layers can be interconnected as well, e.g. for an ease of manufacturing.

Further, also the readout-circuits in the pre-arrays - as in the pre-subarrays - can be interconnected and can be configured to connect suitably the electrodes provided, forming again one readout-circuit. The readout-circuit(s) can also again connect additional vias which can be present and or further electrode bases and/or electrodes, e.g. for forming tunneling electrodes and/or reference electrodes. In the present invention, it is possible to form an array from the present pre-array by e.g. forming pairs of tunneling electrodes on the substrate, e.g. on vias and optionally electrode bases that are already formed in the pre-arrays. Therefore it is also possible to provide a pre-array on which then pairs of tunneling electrodes are formed in a later step, e.g. at a customer's site.

Also, a detector for detecting tunneling currents on a pair or multiple pairs of tunneling electrodes to be formed can already be provided in the present pre-array according to certain embodiments, which is not particularly restricted and which can comprise those known in the state of the art, e.g. from WO 2014048816 A1 and WO 2015010904 A1.

The present setups, pre-subarrays and/or pre-arrays can form a kit together with a component for forming the tunneling electrodes - e.g. a solution or suspension containing material for forming the tunneling electrodes, e.g. at a customer's site, by e.g. application of a suitable voltage to vias configured for contacting tunneling electrodes.

A fifth aspect of the present invention relates to a device for sequencing at least one biopolymer, comprising
the setup of the first aspect;
a pair of tunneling electrodes; and
a detector for detecting a tunneling current through the pair of tunneling electrodes.

In the present device, the pair of tunneling electrodes is not particularly restricted and can be made of any suitable material, e.g. a metal like Au, Pt, Ag, electrically conducting polymers, etc. The pair of tunneling electrodes can be formed at a location suitable for sequencing the at least one biopolymer, i.e. a location to which the at least one biopolymer is moved, e.g. between the at least two main electrodes.

An exemplary embodiment thereof is shown in Fig. 15, where the setup comprises an (optional) auxiliary electrode 2a. In this case the auxiliary electrode 2a is located in the same plane as the main electrodes and the tunneling electrodes 6. Also shown is the pair of main electrodes that is connected by the source providing the longitudinal voltage Vₗ. Furthermore, a capture voltage V_{C} can be applied between the auxiliary electrode 2a and the left main electrode. The setup in the device shown in Fig. 15 is thus similar to the one in Fig. 7. Further, the exemplary device in Fig. 15 shows a pair of tunneling electrodes 6, represented by arrows, between which a tunneling voltage Vₜ can be applied for detecting and sequencing the at least one biopolymer, e.g. a nucleic acid molecule, using the tunneling voltage according to the methods known to the skilled person. In Fig. 15, the nanoporous network layer is excluded for better visibility of the different circuits for applying a voltage.

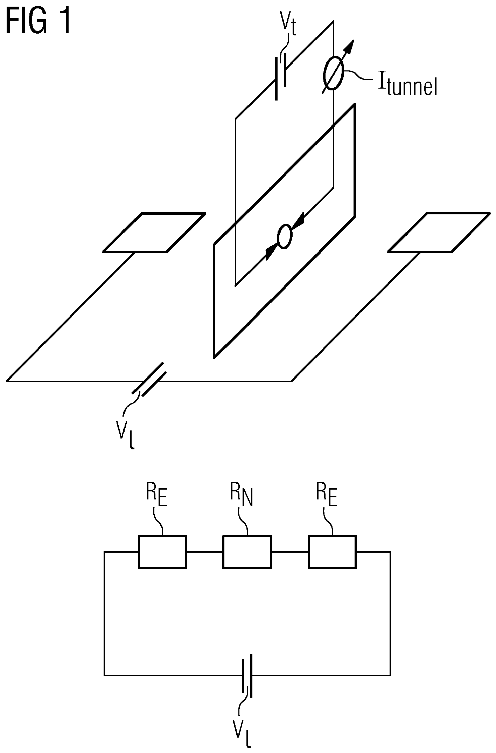

As can be seen from a comparison of Figs. 1 and 15, these show the basic transverse tunneling concept as well as the corresponding planar setup without the nanoporous network layer. In Fig. 15 the catcher-voltage V_{C} and the longitudinal voltage Vₗ are applied one after each other, thus leading to the improved method of the present invention using the present improved setup.

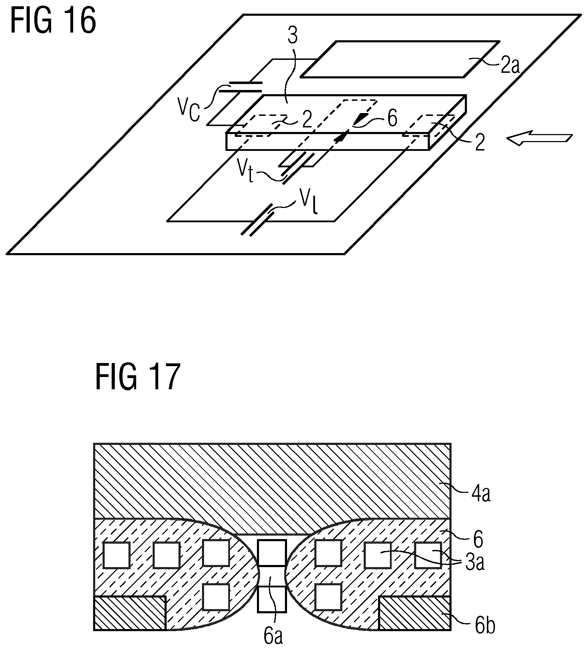

According to certain embodiments, the pair of tunneling electrodes can be provided at least partially in the nanoporous network layer so that the nanopores can provide a further restriction for passing the biopolymers through a gap between the tunneling electrodes. This is shown in more detail in Figs. 16 and 17.

Figs. 16 and 17 show the planar setup of Fig. 15 together with the nanoporous network layer 3, which is particularly visible in Fig. 16 on top of the main electrodes 2. Also a cross-section of the tunneling junction in the setup of Fig. 16 in the direction of the arrow depicted therein, perpendicular to the translocation direction, is shown in Fig. 17. The formation and composition of such a cross-section can also be taken from WO 2015010904 A1. In Fig. 17 especially the base electrodes 6b on which the tunneling electrodes 6 are formed, as well as the gap 6a between the tunneling electrodes, which can also be filled with nanoparticles 3a or similar structures of the nanoporous network layer 3, can be seen.

In Fig. 17, it is also depicted how the hydrophobic liquid 4a which replaces solvent 4 is present on top of the tunneling electrodes 6. The hydrophobic liquid can ensure that the biopolymers are not going over and/or around the tunneling electrodes and are actually passing there through, enabling the sequencing of these biopolymers. Also the junction-cross-section of the tunneling electrodes can be restricted this way.

Further, by an improved design of the solid substrate and/or the nanoporous network layer, as e.g. also described later in connection with Figs. 2, 23, 25, 29 and 30, where e.g. the solid substrate and/or the nanoporous network layer is narrowed down in width next to the tunneling electrodes, e.g. essentially to the width of the tunneling electrodes, it can be further ensured that the at least one biopolymer passes through the tunneling electrode junction by application of the hydrophobic liquid, as the hydrophobic liquid can cover the whole tunneling electrodes and thus ensure that no biopolymer can go around them.

A sixth aspect of the present invention relates to a sub-array for sequencing at least one biopolymer, comprising the pre-subarray of the third aspect;
a multitude of pairs of tunneling electrodes, wherein for each setup of the first aspect a pair of tunneling electrodes is provided; and a detector for detecting tunneling currents through the multitude of pairs of tunneling electrodes.

In the present subarray, the tunneling electrodes can be configured the same way as in the present device, so that the corresponding descriptions also apply to the subarray. Also the detector can be the same as in the present device. In the present subarray, it is possible to provide only one detector which can either sequentially or in parallel detect biopolymers and sequence them at several tunneling electrode pairs, e.g. by multiplexing. Also, it is possible that translocation electrodes, e.g. the two main electrodes, and the pair of tunneling electrodes provided to these translocation electrodes, in a setup can be separately connected to the detector with a single detector circuit, so that in a sub-array just one setup for detection can be selected, e.g. the one with the best efficiency. In an array, these detectors of a sub-array can then be read out using multiplexing, e.g. in parallel, so that a multitude of sub-arrays can be processed simultaneously.

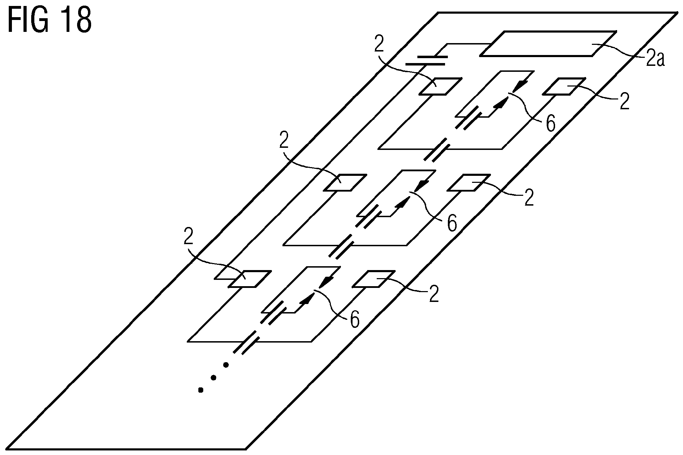

Fig. 18 shows the schematic arrangement of multiple capture / translocation / tunneling units with multiple main electrodes 2 wherein for each pair one acts as catcher electrode, and multiple tunneling electrode pairs 6 in an exemplary subarray, herein supplied by one auxiliary electrode 2a to capture the target biopolymers, e.g. from a larger electrolyte reservoir above the chip over several of the main electrode 2 locations. The tunneling electrodes can then be connected to one detector that is not shown. Also in the figure the readout-circuits are not shown. As indicated by the dots, more than 3 devices, as indicated above, can be present in a sub-array. In this special subarray it is possible that all setup "units" are supplied by one auxiliary electrode to transport target biopolymer molecules to the catcher electrodes of each unit. Thus, in the present subarray, as in the present pre-subarray, it is possible that only one auxiliary electrode is provided. In such instance, the auxiliary electrode can have various different geometries, such as a "bus-structure" around, or a network structure within the array-positions.

A seventh aspect of the present invention is directed to an array, comprising a multitude of sub-arrays of the sixth aspect, or comprising
a pre-array of the fourth aspect;
a multitude of pairs of tunneling electrodes, wherein for each setup of the first aspect a pair of tunneling electrodes is provided; and
at least one detector for detecting tunneling currents through the multitude of pairs of tunneling electrodes.

Also in the present array, the tunneling electrodes can be configured the same way as in the present device, so that the corresponding descriptions also apply to the subarray. Also the detector can be the same as in the present device. In the present array, it is possible to provide a multitude of detectors, e.g. one for each subarray or for each pre-subarray, which can either sequentially or in parallel detect biopolymers and sequence them at several tunneling electrode pairs, e.g. by multiplexing. Thus, it is possible that a multitude of detectors with a multitude of circuits, each e.g. provided for one subarray, can be read out, e.g. in parallel or sequentially, preferably in parallel.

According to certain embodiments, at least one selection switch is contained in the readout-circuit for each pair of tunneling electrodes provided for each setup in a device, subarray, or array of the invention for a connection to one detector.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Example 1

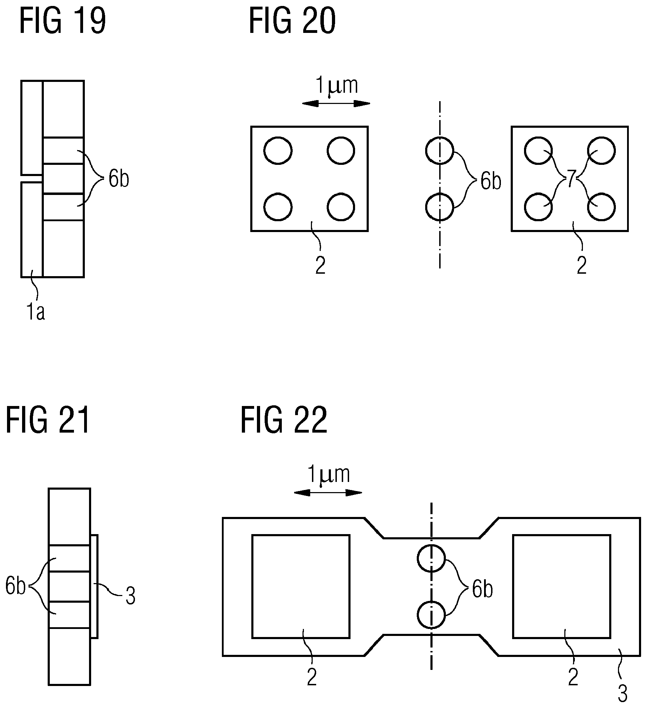

A scheme for an exemplary present setup is given in Figs. 19 to 22.

Fig. 19 therein shows a cross section of a setup wherein vias 6b are inside the upper layer of a substrate, with a CMOS-metal-layer below the vias 6b for contacting. On top of 4 vi-as 7 each two main electrodes 2 can be formed, as shown in Fig. 20, e.g. by a metallization, to form a setup as in Fig. 4 (with the nanoporpous network layer not shown, which can be added after formation of the main electrodes). In such a setup the surface of the solid substrate is not planar as the main electrodes 2 are above the surface, which may lead to a disturbance in the capture and/or translocation due to possible inhomogeneities in the nanoporous network layer. Thus, alternatively, also the vias 7 itself can be used as main electrode, thus forming a kind of "virtual electrode surface" over the vias, which can be e.g. comprise also a field similar to the electrode surface indicated in Fig. 20, thus also forming a "main electrode" 2. In the following, this alternative setup is further used. This setup can be easily processed to produce an essentially flat surface on the solid substrate including the vias 7 as electrodes, e.g. by a polishing process like chemical-mechanical polishing (CMP) .

With usual manufacturing processes, electrode diameters/ lengths and/or distances between electrodes of about 400 nm can be realized. In addition, also two vias 6b, as seen in Fig. 19, can be already formed in the substrate on which tunneling electrodes can be formed later. Fig. 21 shows the vias 6b with the applied nanoporous layer 3 in a cross section indicated in Fig. 22, and Fig. 22 shows the final formation of the nanoporous network layer 3 also over the "virtual surface" of the main electrodes 2, formed by the four vias 7, as indicated in Fig. 20, and over the vias 6b in a shape that narrows near the vias 6b so that the tunneling electrodes will pass beyond the nanoporous network layer 3 upon formation. This can ensure that the whole nanoporous network layer 3 can be covered by a hydrophobic liquid during translocation so that a biopolymer to be analyzed stays inside the nanoporous network layer 3. In this exemplary setup, the nanoporous network layer can be formed with a thickness of about 100 nm, and can have Au-nanobeads embedded therein for better contacting. The main electrodes can be formed e.g. of Pt, Au, W or Cu. In this setup, the electrode surfaces can be planarized by chemical mechanical polishing, e.g. also together with the top substrate layer, e.g. an insulating layer made of e.g. SiO₂ and/or Si₃N₄ before application of the nanoporous network layer. The nanoporous network layer can be e.g. applied by spin-coating of a sol-gel layer, e.g. formed with a metal alcoholate, to which conductive particles, e.g. Au-beads with a diameter of about 5 nm, can be added. After tempering a nanoporous network layer with pores of about 1 to 5 nm size and a thickness of about 100 nm can be formed, which can be structured as shown in Fig. 22 using known semiconductor-technological processes, e.g. with a resist mask and etching.

### Example 2

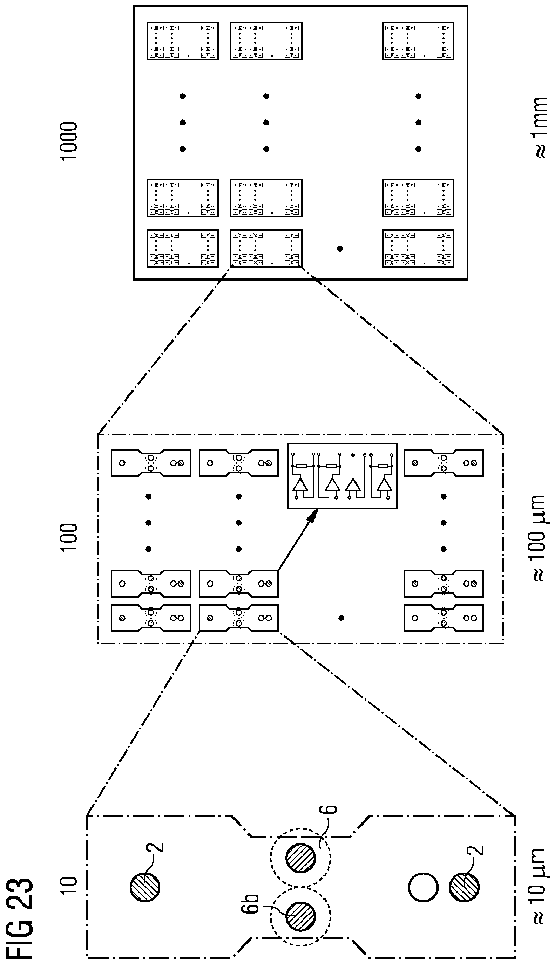

Fig. 23 shows how a present setup 10 with two main electrodes 2 and two vias 6b for tunneling electrodes - on which the tunneling electrodes 6 can be formed as indicated by the circular dashed lines - with a nanoporous network layer formed in a similar fashion as in Example 1 (dashed line around the main electrodes 2 and vias 6b) can be integrated into a pre-subarray 100 comprising a number of setups and one readout-circuit, and how the pre-subarray 100 can be integrated into a pre-array 1000. In the setup 10 a further reference electrode is shown (no numbering), which is not essential but can be used for reference.

As indicated by the bigger dots in the pre-subarray 100 and the pre-array 1000, the number of setups 10, respectively pre-subarrays 100, used therein is not limited.

With pre-subarrays and pre-arrays, respectively subarrays and arrays, it is possible to select efficient setups, respectively devices, for capturing, translocating and eventually detecting and sequencing biopolymers. Also, a parallelization of the processes is possible.

As show in Fig. 23, it is possible that the tunneling electrodes in a device, subarray or array of the invention can be formed at a later point, e.g. at a customer's site.

### Example 3

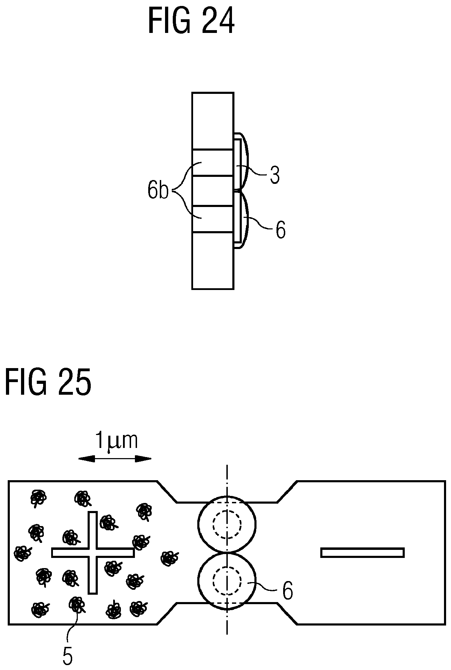

Figs. 24 and 25 show an exemplary present device. Fig. 24 shows a cross-section thereof, similar to the one in Example 1, with the vias 6b contacting the the pair of tunneling electrodes 6 formed on the substrate and the nanoporous network layer 3. As seen in the top-view of Fig. 25, biopolymers 5, here negatively charged nucleic acid molecules, can be captured when the main electrode on the left is applied as positive electrode in the nanoporous network layer, which has a shape as in Example 1. When entering the nanoporous network layer, the biopolymers, e.g. DNA, can get unfolded.

### Examples 4 and 5

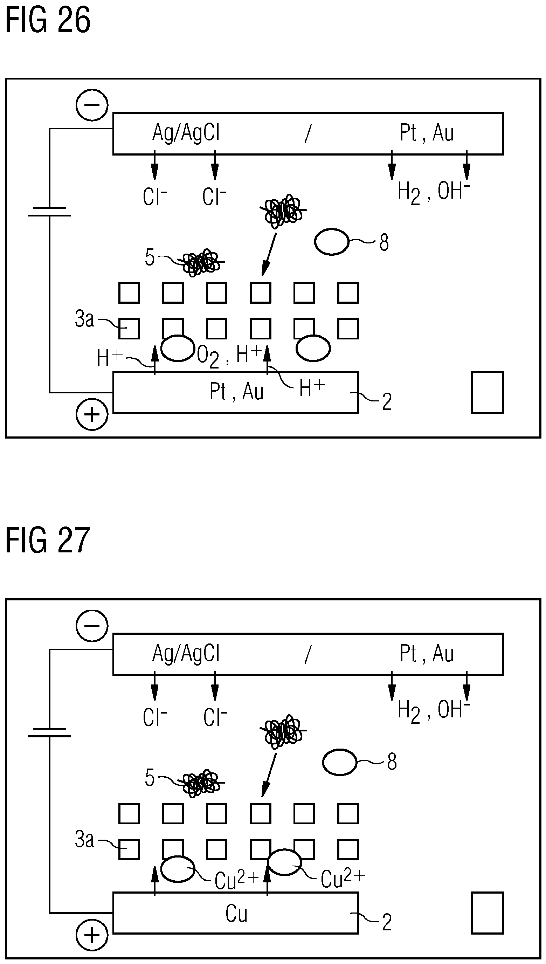

Figs. 26 and 27 show the capture of exemplary biopolymers 5, e.g. DNA, in a setup as shown in Figs. 13 and 14. The biopolymers 5 present in a buffer 8, e.g. a histidine buffer at a pH of 7 - 8, with low ionic strength, for example DNA, can be introduced into the nanoporous network layer, herein exemplified by nanoparticles 3a for better visibility, by applying a suitable voltage between an auxiliary electrode at the top, e.g. made of Ag/AgCl, Pt and/or Au, and a main electrode 2 on the bottom, being made of e.g. Pt and/or Au (Fig. 26, Ex. 4), or Cu (Fig. 27, Ex. 5). Also possible reactions at the electrodes are shown, e.g. the complexation of Cu²⁺ ions by the histidine buffer at the positive electrode.

### Example 6

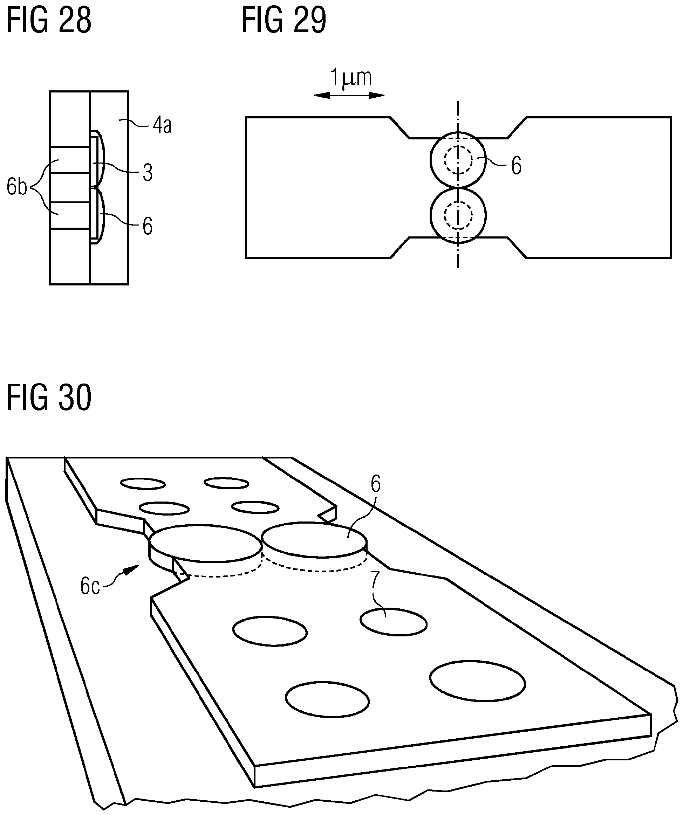

In Figs. 28 to 30 it is shown how biopolymers cannot bypass the device of Example 3 after application of a hydrophobic liquid 4a, e.g. an oil like mineral oil, when the biopolymer are captured inside the nanoporous network layer, with Fig. 28 again showing the cross-section in Fig. 29, and Fig. 29 the top-view with the tunneling electrodes 6 shown in more detail. As is particularly clear from the 3D-view in Fig. 30, the biopolymers cannot by-pass the tunneling electrodes 6 at a location 6c outside thereof after coverage with the hydrophobic liquid and have to pass through the nanoporous network layer.

### Example 7

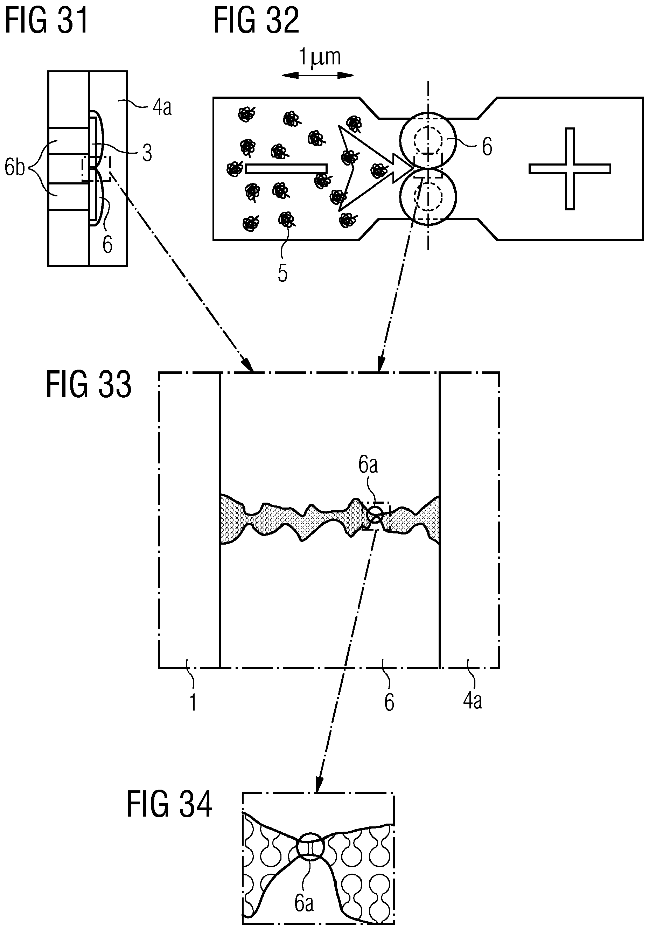

Figs. 31 to 34 show in detail a translocation of biopolymers through a device as shown in Examples 3 and 6.

As shown in Fig. 31, the hydrophobic liquid 4a is applied, so that the captured biopolymers cannot leave the nanoporous network layer 3. As shown in the top-view of Fig. 32, the biopolymers 5, e.g. here nucleic acid molecules having a negative charge like DNA, are moved through the nanoporous network layer 3 towards the tunneling electrodes 6 by applying a negative potential on the main electrode on the left and a positive potential on the main electrode on the right. The biopolymers then can pass through a gap 6a between the tunneling electrodes 6, shown in more detail in Figs. 33 and 34, as the gap is bordered by the solid substrate 1 on one side and the hydrophobic liquid 4a on the other side, through which the biopolymers do not pass, and a pore formation is further facilitated by the nanoporous network layer, as indicated in Fig. 34. This thus forces the biopolymers, e.g. DNA strands, through the electrode gap of the tunneling electrodes. As seen in Fig. 33, the tunneling electrodes can e.g. have an uneven surface after a deposition, e.g. a galvanical deposition of a noble metal like Au. As shown in Fig. 34, a pore between the tunneling electrodes can have suitable conditions for tunneling-based sequencing when a short electrode junction is formed at least at one position and/or when the structure of the nanoporous network layer at the tunneling electrodes aids in the sequencing by further minimizing the pore, as e.g. described in WO 2014048816 A1 and WO 2015010904 A1.

### Examples 8 and 9

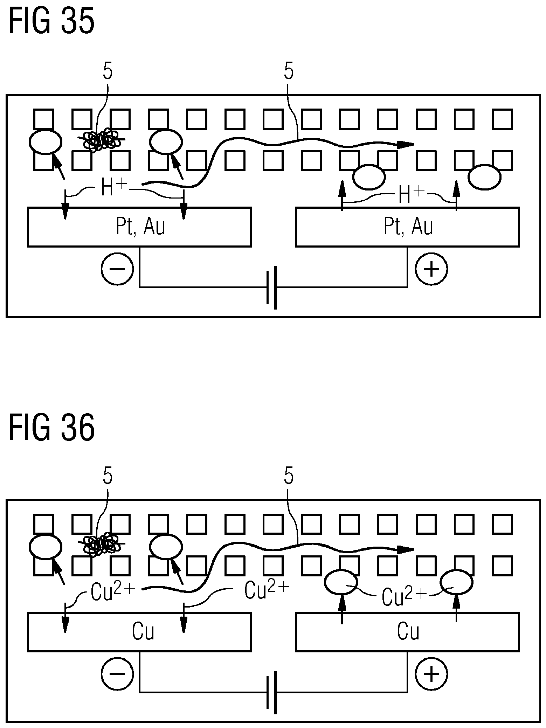

In Figs. 35 (Ex. 8) and 36 (Ex. 9), the translocation of the exemplary biopolymer, DNA, in Examples 4 and 5, respectively, through the nanoporous network layer is shown after applying the respective voltage to the main electrodes, similar to the voltages in Example 7. As can be seen in the Figures, the DNA is elongated through the nanoporous network layer, so that it can be sequenced easily at the tunneling electrodes (not shown). For sequencing, also the polarization can be altered to move the biopolymer, e.g. DNA, back and forth through the tunneling electrodes, respectively nanoporous network layer. Also possible reactions at the electrodes are shown again, e.g. the complexation of Cu²⁺ ions by the histidine buffer at the positive electrode.

With the present invention, a setup- and method-based separation of the capturing and the translocation of an analyte biopolymer can be achieved, allowing the application of dedicated voltages, e.g. high for catch and/or low for translocation.

## Claims

1. A setup for the capture of at least one biopolymer, preferably at least one nucleic acid molecule, and its controlled translocation through a nanoporous network, comprising:
- a flow channel on a solid substrate configured to introduce a solution or suspension of the at least one biopolymer;
- at least two main electrodes on the solid substrate inside the flow channel; and
- a nanoporous network layer in the flow channel on the solid substrate adjacent to and/or on at least one of the two main electrodes;
wherein the flow channel is configured to introduce the at least one biopolymer at a location on an opposite site of at least one of the two main electrodes in relation to the nanoporous network layer.

2. The setup of claim 1, further comprising
- at least one auxiliary electrode, preferably placed on an opposite side of the at least two main electrodes in relation to the nanoporous network layer inside the flow channel.

3. The setup claim 2, wherein at least one of the at least two main electrodes and the at least one auxiliary electrode are placed on opposite sides of the nanoporous network layer facing each other.

4. The setup of any one of the preceding claims, wherein the substrate is a silicon-based or glass-based chip, preferably a CMOS-chip.

5. The setup of any one of the preceding claims, wherein the nanoporous network is hydrophilic.

6. The setup of any one of the preceding claims, wherein the at least two main electrodes, optionally the auxiliary electrode and the nanoporous network layer are placed coplanar on the substrate.

7. A method of capturing at least one biopolymer, preferably at least one nucleic acid molecule, and its controlled translocation through a nanoporous network, comprising:
- providing a setup of any one of claims 1 to 6,
- providing a solution or suspension containing the at least one biopolymer,
- introducing the solution or suspension containing the at least one biopolymer into the setup of any one of claims 1 to 6,
- applying a suitable voltage between at least two electrodes of the setup of any one of claims 1 to 6 for a time sufficient to introduce the at least one biopolymer into the nanoporous network layer,
- replacing the solution or suspension with a hydrophobic liquid, and
- applying a suitable voltage between the at least two main electrodes to translocate the at least one biopolymer inside the nanoporous network layer.

8. The method of claim 7, wherein the at least two electrodes for introducing the at least one biopolymer into the nanoporous network layer are the at least main two electrodes defined in claim 1 or one of the two main electrodes defined in claim 1 and the at least one auxiliary electrode defined in claim 2.

9. The method of claim 7 or 8, wherein the hydrophobic liquid is a mineral oil or a silicon oil.

10. The method of any one of claims 7 to 9, wherein a solvent for the solution or suspension containing the at least one biopolymer is water.

11. A pre-subarray, comprising a multitude of setups of any one of claims 1 to 6 and at least one readout-circuit.

12. A pre-array, comprising a multitude of pre-subarrays of claim 12.

13. A device for sequencing at least one biopolymer, comprising
the setup of any one of claims 1 to 6;
a pair of tunneling electrodes; and
a detector for detecting a tunneling current through the pair of tunneling electrodes.

14. A sub-array for sequencing at least one biopolymer, comprising
a pre-subarray of claim 11;
a multitude of pairs of tunneling electrodes, wherein for each setup of any one of claims 1 to 6 a pair of tunneling electrodes is provided; and
a detector for detecting tunneling currents through the multitude of pairs of tunneling electrodes.

15. An array, comprising a multitude of sub-arrays of claim 14, or comprising
a pre-array of claim 12;
a multitude of pairs of tunneling electrodes, wherein for each setup of any one of claims 1 to 6 a pair of tunneling electrodes is provided; and
at least one detector for detecting tunneling currents through the multitude of pairs of tunneling electrodes.
